## Europäisches Patentamt

## European Patent Office

## Office européen des brevets

(19)

(11) Veröffentlichungsnummer: **0 144 745**
**B1**

(12)

# EUROPÄISCHE PATENTSCHRIFT

(45) Veröffentlichungstag der Patentschrift:
07.01.87

(51) Int. Cl.⁴: **C 07 C 45/50, C 07 C 47/02**

(21) Anmeldenummer: 84113242.6

(22) Anmeldetag: 03.11.84

(54) Verfahren zur Herstellung von Aldehyden.

(30) Priorität: 12.11.83 DE 3341035

(43) Veröffentlichungstag der Anmeldung:
19.06.85 Patentblatt 85/25

(45) Bekanntmachung des Hinweises auf die Patenterteilung:
07.01.87 Patentblatt 87/2

(84) Benannte Vertragsstaaten:
AT BE DE FR GB IT NL SE

(56) Entgegenhaltungen:
EP - A - 0 016 285
DE - A - 3 035 468
DE - A - 3 114 147
DE - A - 3 135 127
DE - A - 3 234 701
DE - B - 2 627 354

(73) Patentinhaber: Ruhrchemie Aktiengesellschaft,
Bruchstrasse 219, D-4200 Oberhausen 11 (DE)

(72) Erfinder: Kalbfell, Heinz, Dipl.-Ing., Eichenstrasse 20,
D-4235 Schermbeck (DE)
Erfinder: Lieder, Bernhard, Siegfriedstrasse 61,
D-4250 Bottrop (DE)
Erfinder: Mercamp, Herbert, Dipl.-Ing., Weyerskamp 18,
D-4220 Dinslaken 3 (DE)

(74) Vertreter: Reichelt, Karl-Heinz, Dr., m. Br. Ruhrchemie
Aktiengesellschaft Abt. PLD Postfach 13 01 60,
D-4200 Oberhausen 13 (DE)

ACTORUM AG

## Beschreibung

Die vorliegende Erfindung betrifft ein verbessertes Verfahren zur Herstellung von Aldehyden durch Hydroformylierung von Olefinen in Gegenwart wasserlöslicher Rhodium-Komplexkatalysatoren.

Katalysatoren der genannten Art sind bekannt und z.B. in der DE-PS 26 27 354 beschrieben. Die Löslichkeit der Rhodiumkomplexverbindungen wird hierbei durch Verwendung trisulfonierter Triarylphosphine als Komplexbestandteil erreicht. Nach einem in der DE 31 35 127 AI offenbarten Prozess setzt man als wasserlösliche Katalysatoren Komplexe von Metallen der Platingruppe, die ein sulfoniertes oder carboxyliertes Phosphin enthalten, ein. Die Umsetzung wird in einem aus organischer und wässriger Phase bestehenden Reaktionsmedium, das ein amphilisches Reagenz enthält, durchgeführt. Ein wesentlicher Vorteil des Einsatzes wasserlöslicher Hydroformylierungskatalysatoren ist ihre einfache Abtrennung vom Reaktionsprodukt nach der Umsetzung, die lediglich eine mechanische Trennung von wässriger und organischer Phase erfordert, d.h. ohne Destillation auskommt, daher keinen zusätzlichen Energieverbrauch hat und eine Anreicherung von Höhersiedern in der Katalysatorphase vermeidet.

Die kontinuierliche Ausgestaltung eines Verfahrens zur Herstellung von Aldehyden durch Umsetzung von Olefinen mit Kohlenmonoxid und Wasserstoff in Gegenwart von Wasser und wasserlöslichen Rhodium-Phosphin-Komplexverbindungen ist in der deutschen Patentanmeldung P 32 34 701.4 beschrieben. Gemäss dieser Arbeitsweise werden die Reaktanten innig vermischt und bei Temperaturen von 90 bis 150° C sowie Drücken von 1 bis 300 bar (100 bis 30.000 kPas) zur Reaktion gebracht. Hierbei wird der Anteil der gasförmigen Bestandteile in der flüssigen Phase auf 5 bis 30 Vol.%, bezogen auf die Mischphase, und das Volum-Verhältnis von wässriger zu organischer Phase auf 1:1 bis 100:1 eingestellt. Bei der Aufarbeitung des Reaktionsproduktes werden zunächst flüssige Phase und gasförmige Phase, sodann die flüssige Phase in wässrige und organische Anteile jeweils ohne vorherige Kühlung getrennt und die Reaktionswärme wird abgeführt.

In der Praxis wird das aus dem Reaktor kommende Reaktionsprodukt, nämlich Aldehyd, in Mischung mit wässriger Katalysatorlösung, nicht umgesetztem Synthesegas und Olefin einem Trenngefäss zugeführt. Dort wird die Gasphase, im wesentlichen Synthesegas und, in Abhängigkeit von ihren Siedepunkten, auch Olefin, aus dem Olefin gebildeter und mit dem Olefin eingetragener gesättigter Kohlenwasserstoff und Aldehyd von den Flüssigprodukten getrennt. Die Gasphase wird in den Reaktor rezirkuliert, eine kleine Menge als Abgas ausgetragen. Die Flüssigkeit trennt man in das rohe organische Reaktionsprodukt und die wässrige, den Katalysator enthaltende Phase, die in den Reaktor zurückgeführt wird. Das organische Reaktionsprodukt wird zweckmässig einer Strippkolonne aufgegeben und im Gegenstrom zu einer Synthesegasteilmenge geführt. Dabei belädt sich das Synthesegas unter anderem mit dem im Rohprodukt gelösten Olefin. Aus der Strippkolonne gelangt das Oxorohprodukt in die Destillation und wird hier in seine Bestandteile zerlegt.

Entscheidend für eine wirtschaftliche Ausgestaltung des Prozesses ist die Vermeidung von Katalysator-, Produkt- und Wärmeverlusten.

Katalysatorverluste werden vor allem dadurch verursacht, dass Katalysator in geringer Menge mit dem Rohprodukt ausgetragen wird und, weil er in niedriger Konzentration vorliegt, nicht zurückgewonnen werden kann.

Bei der Entspannung des mit gelöstem Synthesegas gesättigten Produktstromes nach der Strippkolonne auf Atmosphärendruck wird mit dem Abgas auch Reaktionsprodukt ausgeschleust, das somit ebenfalls verlorengeht.

Schliesslich wird Reaktionswärme mit dem Abgas und über das nach der Strippkolonne anfallende Rohprodukt abgeführt.

Es bestand daher die Aufgabe, einen Hydroformylierungsprozess mit wasserlöslichen Katalysatoren zu entwickeln, der die im aufgezeigten Stoff- und Energieverluste vermeidet.

Die Erfindung besteht in einem Verfahren zur Herstellung von Aldehyden durch Umsetzung von Olefinen mit Kohlenmonoxid und Wasserstoff in Gegenwart von Wasser und wasserlöslichen Rhodium-Phosphin-Komplexverbindungen als Katalysator bei 90 bis 150° C und Drücken von 1 bis 300 bar, Trennung des Reaktionsproduktes in flüssige Phase und gasförmige Phase und der flüssigen Phase in wässrige und organische Anteile jeweils ohne vorherige Kühlung und Rückführung des wässrigen, den Katalysator enthaltenden Anteils der flüssigen Phase in den Reaktor. Es ist dadurch gekennzeichnet, dass der organische Anteil der flüssigen Phase auf 20-35° C abgekühlt anschliessend unter Bildung von Abgas entspannt und in einem Trenngefäss in eine organische und eine wässrige Phase getrennt und darauf die organische Phase destilliert wird.

Die erfindungsgemässe Arbeitsweise eignet sich zur Überführung von Olefinen mit 2 bis 15 Kohlenstoffatomen in die um ein Kohlenstoffatom reicheren Aldehyde, die durch Hydrierung in die entsprechenden Alkohole umgewandelt werden können. Reaktionspartner der Olefine ist Synthesegas, das Kohlenmonoxid und Wasserstoff zweckmässigerweise im Vol.-Verhältnis 1:1 enthält. Es ist möglich, dieses Verhältnis zur Erreichung bestimmter Effekte, z.B. zur Steigerung der Reaktionsgeschwindigkeit, zu variieren.

Die Umsetzung der Ausgangsstoffe im Reaktor erfolgt bei Temperaturen von 90 bis 150° C und Drücken von 1 bis 300 bar (entsprechend 100 bis 30.000 kPas) in einem aus flüssiger und gasförmiger Phase bestehenden System. Hierbei besteht die flüssige Phase aus zwei ineinander nicht oder nur sehr wenig löslichen Komponenten, der wässrigen Katalysatorlösung, gegebenenfalls dem flüssigen Olefin und dem flüssigen organischen Reaktionsprodukt, das auch noch ein Lösungsmittel enthalten kann.

Als Katalysatoren werden komplexe Verbindungen des Rhodiums eingesetzt, die neben Kohlenmonoxid und Wasserstoff sulfonierte oder carboxylierte Phosphine enthalten. Derartige Phosphine leiten sich insbesondere von Triarylphosphinen ab, wobei unter Arylrest vorzugsweise der Phenylrest und der Napthylrest verstanden wird. Es ist nicht erforderlich, dass alle drei Arylreste Sulfonsäuregruppen oder Carboxylgruppen tragen. Es hat sich gezeigt, dass bereits eine Sulfonsäuregruppe oder eine Carboxylgruppe im Phosphinmolekül der Komplexverbindung eine ausreichende Wasserlöslichkeit verleiht. Der Katalysator kann dem Reaktionsgemisch präformiert zugesetzt werden. Es ist aber auch möglich, ihn in situ zu bilden. Üblicherweise setzt man Rhodium in einer Menge von 50 bis 800 ppm, bezogen auf die wässrige Katalysatorlösung, zu. Das sulfonierte oder carboxylierte Triarylphosphin muss, bezogen auf den Rhodiumkomplex, im Überschuss vorhanden sein. Besonders bewährt hat es sich, je g-Atom Rhodium 10 bis 100 g Moleküle sulfoniertes oder carboxyliertes Phosphin zuzusetzen.

Massgebend für den Ablauf der Reaktion ist, dass die wässrige Phase mit den gasförmigen Reaktanten Kohlenmonoxid, Wasserstoff und in Abhängigkeit von Reaktionsbedingungen und Molekülgrösse, gasförmigem Olefin gesättigt ist. Um dies zu erreichen, muss eine möglichst grosse Berührungsfläche zwischen der flüssigen – also aus wässriger und organischer Komponente bestehenden – und der gasförmigen Phase herbeigeführt werden, so dass der Anteil der gasförmigen Bestandteile in der flüssigen Phase auf 5 bis 30 Vol.%, bezogen auf die Mischphase, eingestellt wird. Hierzu führt man z.B. die gasförmigen Ausgangsstoffe dem Reaktorinhalt unter intensivem Rühren zu oder man leitet sie über entsprechende Verteilungsvorrichtungen in den flüssigen Reaktorinhalt ein. Geeignet sind z.B. Siebböden oder Fritten. Es ist auch möglich, Rührung und Verteilung der gasförmigen Reaktionspartner miteinander zu kombinieren, beispielsweise durch Verwendung eines Begasungsrührers.

Das Volumverhältnis von wässriger zu organischer Phase beträgt 1:1 bis 100:1, vorzugsweise 10:1 bis 100:1. Zur Einstellung des genannten Volumenverhältnisses kann man z.B. einen solchen Teil des Reaktionsgemisches aus dem Reaktor ausschleusen und einer Phasentrennung unterwerfen, dass sich nach Rückführung der wässrigen Phase das gewünschte Volumverhältnis im Reaktor ausbildet.

Nach einer anderen Ausführungsform kann die Phasentrennung auch innerhalb des Reaktors in einer Beruhigungszone vorgenommen werden.

Die Phasentrennung erfolgt in jedem Fall ohne vorherige Kühlung des Reaktionsgemisches. Durch diese Massnahme wird erreicht, dass die gasförmigen Olefine in den unter den gegebenen Bedingungen flüssigen Bestandteilen des Reaktionsgemisches nur in kleinen Mengen gelöst und mit dem Reaktionsprodukt ausgetragen werden.

Der wässrige Anteil der flüssigen Phase wird, gegebenenfalls nach Ersatz von Katalysatorverlusten, wieder dem Reaktor zugeleitet.

Das Reaktionsprodukt, d.h. der organische Anteil der flüssigen Phase, wird erfindungsgemäss auf Temperaturen von 20 bis 35° C abgekühlt.

Nach einer bevorzugten Ausführungsform der neuen Arbeitsweise erfolgt die Abkühlung des Reaktionsproduktes durch Wärmeaustausch mit Synthesegas, also dem Kohlenmonoxid/Wasserstoff-Gemisch und/oder dem Einsatzolefin. Sowohl Synthesegas als auch Olefin werden anschliessend dem Reaktor zugeführt.

Besonders zweckmässig ist es, die Abkühlung des Reaktionsproduktes in zwei Stufen vorzunehmen. Bei dieser Variante des erfindungsgemässen Prozesses hat es sich bewährt, das Produkt in der ersten Stufe mit Kohlenmonoxid und Wasserstoff in einer Strippkolonne auf 70 bis 90° und in der zweiten Stufe mit dem Einsatzolefin in einem der Strippkolonne nachgeschalteten Wärmeaustauscher auf 20 bis 35° C zu kühlen.

Das abgekühlte Reaktionsprodukt wird anschliessend einem Zwischenentspannungsgefäss zugeführt, in dem der Druck vermindert wird, üblicherweise auf 1 bis 10 bar. Durch Behandlung des Rohproduktes mit Synthesegas in der Strippkolonne werden gelöstes gasförmiges Olefin und gelöster gesättigter Kohlenwasserstoff (der durch Hydrierung aus dem Olefin entstanden ist bzw. mit dem Einsatzolefin eingetragen wurde) aus dem Rohprodukt nahezu vollständig entfernt.

Von besonderer Bedeutung ist, dass das Rohprodukt in einem der Strippkolonne nachgeschalteten Wärmeaustauscher weiter abgekühlt wird. Dadurch wird die Sättigungskonzentration des Wassers in der organischen Phase überschritten und gelöstes Wasser aus der Lösung abgeschieden. Es lässt sich in einfacher Weise mechanisch und ohne Destillation von der organischen Phase trennen. Die Abscheidung des überwiegenden Teils des gelösten Wassers aus dem Rohprodukt ist gleichbedeutend mit einer Extraktion im Rohprodukt enthaltener wasserlöslicher Bestandteile durch Wasser. Bei diesen wasserlöslichen Bestandteilen handelt es sich insbesondere um Rhodiumsalze und sulfonierte oder carboxilierte Arylphosphine. Der Extraktionseffekt kann dadurch verstärkt werden, dass die Wasserfraktion der Aldehyddestillation dem Rohprodukt vor der Phasentrennung zugemischt wird. Zusammen mit dem zur Abtrennung der Wasserphase aus dem gekühlten und teilweise entspannten Rohprodukt vorgesehenen Trenngefäss stellt die Wasserextraktion sehr wirksame Vorkehrungen zur Vermeidung von Katalysatorverlusten dar.

Nach einer bevorzugten Ausführungsform des erfindungsgemässen Verfahrens wird die wässrige Phase in den Reaktor zurückgeführt.

Entsprechend der Temperatur und der Zusammensetzung des Rohproduktes enthält das bei der Verminderung des Druckes entstehende Entspannungsgas im wesentlichen Aldehyde. Um diese Aldehyde möglichst weitgehend zurückzugewinnen, wird das Gas mit Kaltwasser von 5 - 10° C gewaschen. Auf diesem Wege erzielt man eine erhebliche Minderung der Wertproduktverluste. Das Waschwaser wird nach einer zweckmässigen Ausführungsform des erfindungsgemässen Verfahrens dem Sumpf der Strippkolonne zugeleitet.

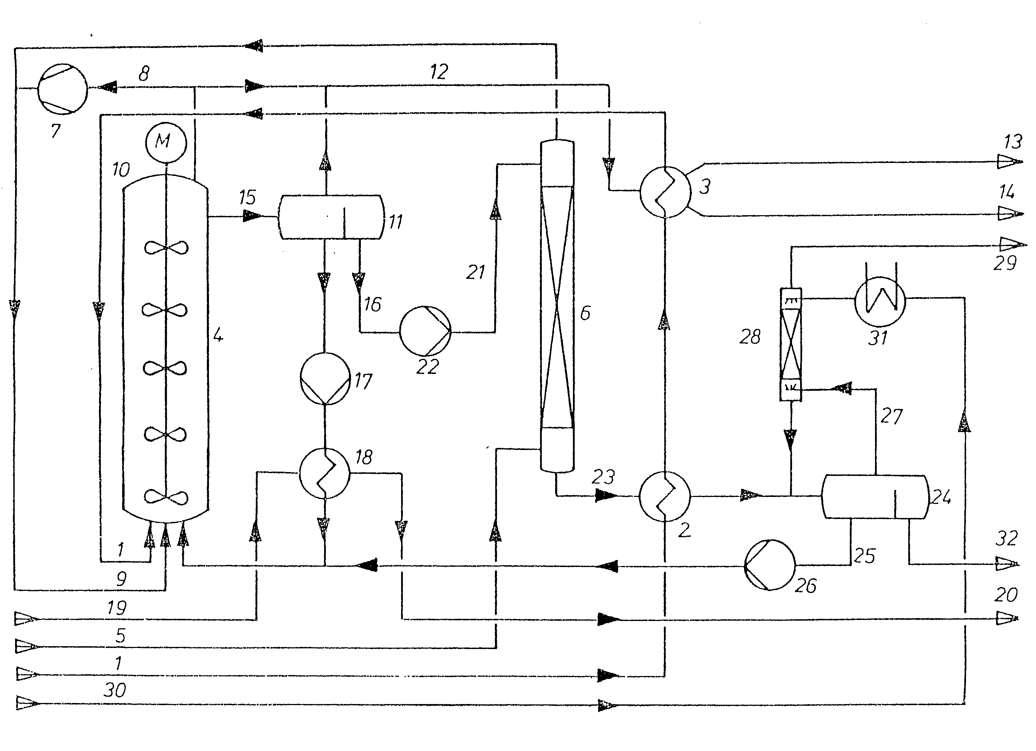

Die Durchführung des erfindungsgemässen Verfahrens ist in der Zeichnung schematisch dargestellt.

Das Einsatzolefin wird über eine Leitung 1 in einem Wärmeaustauscher 2 durch das Rohprodukt und in einem Wärmetauscher 3 durch das Abgas aufgeheizt und darauf einem Reaktor 4 zugeleitet.

Über eine Leitung 5 gelangt Synthesegas in eine Strippkolonne 6 und durchströmt sie von unten nach oben. Es wird dabei aufgeheizt und nimmt im Rohprodukt gelöstes flüchtiges Olefin auf. Kreislaufgas gelangt über eine Leitung 8 in einen Verdichter 7, wird komprimiert und zusammen mit dem vorgewärmten Synthesegas über eine Leitung 9 ebenfalls dem Reaktor 4 zugeführt.

Am Kopf des Reaktors 4 sowie aus einem nachgeschalteten Phasentrenner 11 wird das überschüssige Gas abgeleitet und über die Leitung 8 in den Gaskreislauf und über eine Leitung 12 in den Abgasstrom aufgeteilt. Das Abgas wird im Wärmetauscher 3 durch das Einsatzolefin gekühlt und teilweise kondensiert. Restliches Gas wird über eine Leitung 13, das Abgaskondensat über eine Leitung 14 der weiteren Verwendung zugeführt.

Neben geringen Mengen gasförmiger Bestandteile enthält der über eine Leitung 15 zu einem Trenngefäss 11 abgezogene Überlauf Rohprodukt und Katalysatorlösung. Die am Sumpf des Trenngefässes über eine Leitung 16 austretende Katalysatorlösung wird über eine Kreislaufpumpe 17 und einen Dampferzeuger 18 zum Reaktor 4 zurückgeführt.

Im Dampferzeuger 18 wird die Reaktionswärme dazu genutzt, aus über eine Leitung 19 eingespeistem Kondensat Dampf zu erzeugen, der über eine Leitung 20 abgezogen wird.

Die organische Phase wird über eine Leitung 21 und über eine Pumpe 22 der Strippkolonne 6 zugeleitet.

Das am Sumpf der Strippkolonne austretende Produkt gelangt über eine Leitung 23 zum Wärmeaustauscher 2 und entspannt sich in ein Trenngefäss 24. Die abgetrennte Wasserphase wird über eine Leitung 25 und eine Pumpe 26 dem Reaktor 4 wieder zugeführt.

Das Entspannungsgas strömt über eine Leitung 27 einem Wäscher 28 zu und verlässt diesen über eine Leitung 29 als Abgas. Die Temperatur der aus der Destillation über eine Leitung 30 zurückgeführten Wasserphase wird in einem Kaltwasserkühler 31 gesenkt und über den Wäscher dem Trenngefäss 24 zugeführt. Über eine Leitung 32 fliesst mit dem Systemdruck des Trenngefässes Wertprodukt der nachgeschalteten Destillation oder dem Zwischentank zu.

*Beispiel :*

Propylen von 95% Reinheit wird mit Synthesegas (Molverhältnis $H_2:CO = 1:1$) bei 50 bar Druck und 120° C in Gegenwart einer Rhodiumtriphenylphosphintrisulfonatverbindung als Katalysator zur Reaktion gebracht.

Das Reaktionsprodukt wird von den gasförmigen Anteilen befreit und in eine wässrige und eine organische Phase aufgetrennt. Die organische Phase, das Oxo-Rohprodukt, wird ungekühlt am Kopf der Strippkolonne aufgegeben. Es enthält n- und i-Butanal, daneben gelöst noch etwa 14 Gew.% Propylen, etwa 9 Gew.% Propan sowie 5,3 Gew.% Wasser.

Das Oxo-Rohprodukt wird mit Synthesegas, das als Folge der Kompression eine Temperatur von etwa 80° C besitzt, ohne Wärmezufuhr von aussen in der Strippkolonne behandelt. Es kühlt sich durch die nahezu vollständige Verdampfung der $C_3$-Kohlenwasserstoffe und einer kleinen Menge Butanal von 120° C auf etwa 70° C ab. Im Oxo-Rohprodukt bleiben nur etwa 0,1 Gew.% $C_3$-Kohlenwasserstoffe gelöst.

Das am Sumpf der Strippkolonne austretende Rohprodukt wird durch Einsatzolefin auf etwa 40° C weiter heruntergekühlt. Dabei scheidet sich eine wässrige Phase ab, die Katalysator gelöst enthält. Im nachgeschalteten Trenngefäss wird diese wässrige Lösung abgetrennt und in den Katalysatorkreislauf zurückgeführt. Dadurch wird etwa 90 Gew.% der mit dem Rohprodukt aus dem Reaktionssystem ausgetragenen Katalysatormenge zurückgewonnen.

Das nach Passieren der Strippkolonnne im Oxo-Rohprodukt gelöste Synthesegas wird durch Entspannung auf einen Druck von 3 bar weitgehend freigesetzt und in einer Waschkolonne mit Wasser gekühlt. Zweckmässig verwendet man das Kühlwasser zur Ergänzung von Wasserverlusten der Katalysatorlösung.

## Patentansprüche

1. Verfahren zur Herstellung von Aldehyden durch Umsetzung von Olefinen mit Kohlenmonoxid und Wasserstoff in Gegenwart von Wasser und wasserlöslichen Rhodium-Phosphin-Komplexverbindungen als Katalysator bei 90 bis 150° C und Drücken von 1 bis 300 bar, wobei der Anteil der gasförmigen Bestandteile in der flüssigen Phase auf 5 bis 30 Vol.-%, bezogen auf die Mischphase, eingestellt wird und das Volumverhältnis von wässriger zu organischer Phase 1:1 bis 100:1 beträgt, Trennung des Reaktionsproduktes in flüssige Phase und gasförmige Phase und der flüssigen Phase in wässrige und organische Anteile, jeweils ohne vorherige Kühlung, und Rückführung des wässrigen, den Katalysator enthaltenden Anteils der flüssigen Phase in den Reaktor, dadurch gekennzeichnet, dass der organische Anteil der flüssigen Phase auf 20 bis 35° C abgekühlt anschliessend unter Bildung von Abgas entspannt, in einem Trenngefäss in eine organische und eine wässrige Phase getrennt und darauf die organische Phase destilliert wird.

2. Verfahren nach Anspruch 1, dadurch gekennzeichnet, dass die Abkühlung des organischen Anteils der flüssigen Phase mit Kohlenmonoxid und Wasserstoff und/oder dem Einsatzolefin erfolgt.

3. Verfahren nach Anspruch 1 und 2, dadurch gekennzeichnet, dass die Abkühlung des organischen Anteils der flüssigen Phase in zwei Stufen erfolgt.

4. Verfahren nach Anspruch 3, dadurch gekennzeichnet, dass in der ersten Stufe der organische Anteil der flüssigen Phase mit Kohlenmonoxid und Wasserstoff in einer Strippkolonne bis auf 70 bis 90° C gekühlt wird.

5. Verfahren nach Anspruch 3, dadurch gekennzeichnet, dass in der zweiten Stufe der organische Anteil der flüssigen Phase mit dem Einsatzolefin in einem Wärmeaustauscher auf 20 bis 35° C abgekühlt wird.

6. Verfahren nach Anspruch 1 bis 5, dadurch gekennzeichnet, dass die bei Abkühlung des organischen Anteils der flüssigen Phase und bei Entspannung gebildete wässrige Phase in den Reaktor zurückgeführt wird.

7. Verfahren nach Anspruch 4, dadurch gekennzeichnet, dass das bei der Entspannung der flüssigen Phase gebildete Abgas mit Kaltwasser gewaschen und das Waschwasser zum Sumpf der Strippkolonne zurückgeführt wird.

## Claims

1. A process for the preparation of aldehydes by the reaction of olefins with carbon monoxide and hydrogen in the presence of water and watersoluble rhodium-phosphine complex compounds as catalysts at temperatures of 90 to 150° C and pressures of 1 to 300 bar whereby the proportion of the gaseous components in the liquid phase is set at 5 to 30% by volume related to the mixed phase and the volume ratio of aqueous to organic phase is 1:1 to 100:1, separation of the reaction product into liquid and gaseous phases and the liquid phase into aqueous and organic parts, in each case without prior cooling and return of the aqueous part of the liquid phase containing the catalyst to the reactor, characterised in that the organic part of the liquid phase is cooled to 20 to 35° C, subsequently pressure-relieved with consequent formation of waste gas, separated in a separating vessel into an organic and aqueous phase whereupon the organic phase is then distilled.

2. A process according to claim 1, characterised in that the organic part of the liquid phase is cooled with carbon monoxide and hydrogen and/or the feed olefin.

3. A process according to claims 1 and 2, characterised in that the organic part of the liquid phase is cooled in two stages.

4. A process according to claim 3, characterised in that in the first stage the organic part of the liquid phase is cooled to 70 to 90° C with carbon monoxide and hydrogen in a stripping column.

5. A process according to claim 3, characterised in that in the second stage the organic part of the liquid phase is cooled to 20 to 35° C with the feed olefin in a heat exchanger.

6. A process according to claims 1 to 5, characterised in that the aqueous phase formed during the cooling of the organic part of the liquid phase and pressure-relief is returned to the reactor.

7. A process according to claim 4, characterised in that the waste gas formed when the liquid phase is pressure-relieved is washed with cold water and the wash water is returned to the bottom of the stripping column.

## Revendications

1. Procédé pour la fabrication d'aldéhydes par réaction d'oléfines avec le monoxyde de carbone et l'hydrogène en présence d'eau et de composés complexes rhodium-phosphines solubles dans l'eau comme catalyseur à 90-150° C sous des presions de 1 à 300 bars, la fraction des constituants gazeux dans la phase liquide étant ajustée à 5-30% en volume, par rapport à la phase mixte et le rapport en volume de la phase aqueuse à la phase organique étant de 1:1 - 100:1, séparation du produit de réaction en phase liquide et phase gazeuse et de la phase liquide en fraction aqueuse et fraction organique, chaque fois sans refroidissement préalable, et recyclage dans le réacteur de la fraction aqueuse de la phase liquide contenant le catalyseur, caractérisé en ce que la fraction organique de la phase aqueuse est refroidie à 20-35° C, ensuite détendue avec formation de gaz résiduaire, séparée dans un récipient séparateur en une phase organique et une phase aqueuse et ensuite la phase organique est distillée.

2. Procédé selon la revendication 1, caractérisé en ce que le refroidissement de la fraction organique de la phase aqueuse a lieu avec du monoxyde de carbone et de l'hydrogène et/ou avec l'oléfine utilisée.

3. Procédé selon les revendications 1 et 2, caractérisé en ce que le refroidissement de la fraction organique de la phase liquide a lieu en deux stades.

4. Procédé selon la revendication 3, caractérisé en ce que la fraction organique de la phase liquide est refroidie dans le premier stade à 70-90° C dans une colonne d'extraction par du monoxyde de carbone et de l'hydrogène.

5. Procédé selon la revendication 3, caractérisé en ce que la fraction organique de la phase liquide est refroidie dans le second stade à 20-35° C dans un échangeur de chaleur par l'oléfine utilisée.

6. Procédé selon les revendications 1 à 5, caractérisé en ce que la phase aqueuse formée par refroidissement de la fraction organique de la phase liquide et par détente est recyclée dans le réacteur.

7. Procédé selon la revendication 4, caractérisé en ce que le gaz résiduaire formé dans la détente de la phase aqueuse est lavé à l'eau froide et l'eau de lavage est renvoyée au pied de la colonne d'extraction.